# EUROPEAN PATENT APPLICATION

(11) **EP 4 487 945 A1**
(43) Date of publication of application: **08.01.2025**
(21) Application number: 22928870.9
(22) Date of filing: 01.11.2022
(51) Int. Cl.: B01J 8/06, B01D 53/22, C07C 29/152, C07C 31/04

(54) **REACTOR MODULE AND SEPARATION MEMBRANE MODULE**

(30) Priority: 28.02.2022 JP 2022029392
(71) Applicant: NGK Insulators, Ltd., Nagoya-shi, Aichi 467-8530 (JP)
(72) Inventor: NAKAGAWA, Kosuke, Nagoya-shi, Aichi 467-8530 (JP); IIDA, Kazuki, Nagoya-shi, Aichi 467-8530 (JP); KAN, Hirofumi, Nagoya-shi, Aichi 467-8530 (JP); TORII, Atsushi, Nagoya-shi, Aichi 467-8530 (JP); SHIOMI, Makoto, Nagoya-shi, Aichi 467-8530 (JP); SHIBAGAKI, Yukinari, Nagoya-shi, Aichi 467-8530 (JP); SHIMIZU, Katsuya, Nagoya-shi, Aichi 467-8530 (JP)
(74) Representative: Müller-Boré & Partner Patentanwälte PartG mbB
(86) International application number: PCT/JP2022/040915
(87) International publication number: WO 2023/162352

(57) **Abstract**

A reactor module (2) includes a housing (3) and a plurality of reactors (1) arranged about a center axis (AX) in the housing (3). In a cross section taken along a radial direction perpendicular to the center axis (AX), shortest distances (D1) between the housing (3) and the reactors (1) are equivalent to each other.

## Description

### TECHNICAL FIELD

The present invention relates to a reactor module and a separation membrane module.

### BACKGROUND ART

Conventionally, reactor modules used in a conversion reaction in which a source gas containing hydrogen and carbon oxide is converted into a liquid fuel (e.g., methanol or ethanol) have been developed.

Patent Document 1 discloses a reactor module including a plurality of reactors housed in a housing. The reactors are tube-type reactors.

### CITATION LIST

### Patent Literature

Patent Literature 1: JP 2018-008940A

### SUMMARY

### TECHNICAL PROBLEM

In a reactor module disclosed in Patent Literature 1, the distance between the reactor and the housing is different for each reactor, and therefore the reactors are in a non-uniform thermal environment. Accordingly, among the plurality of reactors, there may be reactors that are excessively heated or cooled, and as a result of that, the conversion efficiency of those reactors may be relatively lower, or the lifespan of those reactors may be shorter.

Also, in a separation membrane module provided with separation filters, when heating or cooling a separation membrane for allowing a desired component contained in a mixed fluid to pass therethrough, if there is a separation filter that has not been sufficiently heated or cooled, or a separation filter that has been excessively heated or cooled, the separation performance may deteriorate and the lifespan may be shorter.

An object of the present invention is to provide a reactor module and a separation membrane module capable of obtaining a more uniform thermal environment.

### SOLUTION TO PROBLEM

A reactor module according to a first aspect of the present invention includes a housing, and a plurality of reactors arranged about a predetermined axis in the housing. In a cross section along a radial direction perpendicular to the predetermined axis, shortest distances between the housing and the plurality of reactors are equivalent to each other.

A reactor module according to a second aspect of the present invention is the reactor according to the first aspect, in which the plurality of reactors are arranged on one analogous line analogous to an outline of an inner peripheral surface of the housing.

A reactor module according to a third aspect of the present invention is the reactor according to the first aspect, in which the housing is hollow, and the plurality of reactors include a plurality of first reactors arranged on a first analogous line analogous to an outline of an inner peripheral surface of the housing, and a plurality of second reactors arranged on a second analogous line analogous to the outline of the inner peripheral surface of the housing and surrounding the first analogous line.

A reactor module according to a fourth aspect of the present invention is the reactor according to any one of the first to third aspects, in which an external shape of the housing is a circular columnar shape or an elliptical columnar shape centered on the predetermined axis, and an external shape of each of the plurality of reactors is a circular columnar shape or an elliptical columnar shape.

A reactor module according to a fifth aspect of the present invention is the reactor according to any one of the first to fourth aspects, in which the reactors each include a separation membrane permeable to a product of conversion reaction for converting a source gas containing hydrogen and carbon oxide into a liquid fuel, a first flow path located on non-permeation side of the separation membrane, and a second flow path located on permeation side of the separation membrane, in each of the reactors, the second flow path includes a first opening and a second opening in respective surfaces of the reactor, the first openings are in communication with a non-outlet-side space in the housing, the second openings are in communication with an outlet-side space in the housing, and the non-outlet-side space is isolated from the outlet-side space.

The reactor module according to a sixth aspect of the present invention is the reactor according to the fifth aspect, first ventilation resistance members disposed in the non-outlet-side space and respectively surrounding the first openings of the reactors, and the first flow resistance members provide flow resistance to a sweep gas flowing into the first openings from the non-outlet-side space.

A reactor module according to a seventh aspect of the present invention is the reactor according to the sixth aspect, further including second flow resistance members disposed in the outlet-side space and respectively surrounding the second openings of the reactors, in which the second flow resistance members provide flow resistance to a sweep gas flowing out from the second openings to the outlet-side space, and a flow resistance of either one of the first flow resistance member and the second flow resistance member is larger than a flow resistance of another one of the first flow resistance member and the second flow resistance member.

A separation membrane module according to an eighth aspect of the present invention is the reactor according to the first aspect includes a housing, and a plurality of separation filters arranged about a predetermined axis in the housing and requiring heating or cooling. In a cross section taken along a radial direction perpendicular to the predetermined axis, shortest distances between the housing and the plurality of separation filters are equivalent to each other.

### ADVANTAGEOUS EFFECTS

According to the present invention, a reactor module and a separation membrane module capable of obtaining a more uniform thermal environment can be provided.

### BRIEF DESCRIPTION OF DRAWINGS

FIG. 1 is a transparent side view of a reactor module according to a first embodiment.
FIG. 2 is a cross-sectional view taken along line X-X in FIG. 1.
FIG. 3 is a perspective view of a reactor according to the first embodiment.
FIG. 4 is a cross-sectional view taken along line A-A in FIG. 3.
FIG. 5 is a cross-sectional view taken along line B-B in FIG. 3.
FIG. 6 is a cross-sectional view taken along line C-C in FIG. 4.
FIG. 7 is a cross-sectional view of a reactor module according to a second embodiment.
FIG. 8 is a cross-sectional view of a reactor module according to Modification 6.
FIG. 9 is a side view of a flow resistance member according to Modification 6.
FIG. 10 is a top view of a discharge pipe according to Modification 6.

### DESCRIPTION OF EMBODIMENTS

### 1. First Embodiment

### Reactor Module 2

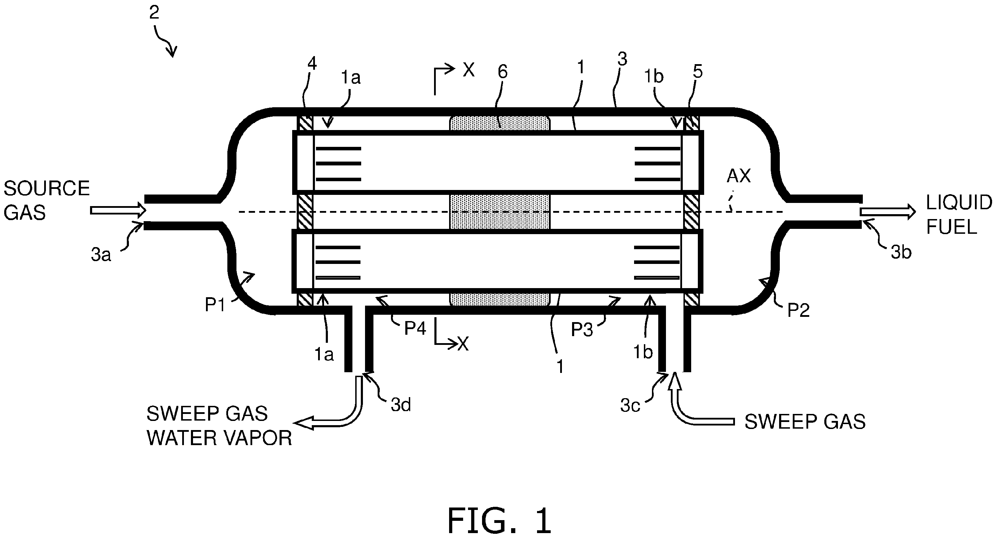

A reactor module 2 according to a first embodiment will be described. FIG. 1 is a transparent side view of the reactor module 2.

As shown in FIG. 1, the reactor module 2 includes a plurality of reactors 1, a housing 3, a first sealing portion 4, a second sealing portion 5, and a flow stopper 6.

### Reactor 1

The reactors 1 are housed in the housing 3. In the present embodiment, the external shape of the reactors 1 is a circular columnar shape.

The reactors 1 are so-called membrane reactors for converting a source gas into a liquid fuel. The source gas contains at least hydrogen and carbon dioxide. The source gas may contain carbon monoxide. The source gas may be a so-called synthesis gas (syngas). The liquid fuel is a fuel that is in a liquid state at normal temperature and normal pressure or a fuel that can be liquefied at normal temperature in a pressurized state. Examples of fuels that are in a liquid state at normal temperature and normal pressure include methanol, ethanol, a liquid fuel represented by CₙH₂₍ₘ₋₂ₙ₎ (m is an integer smaller than 90, and n is an integer smaller than 30), and a mixture of these. Examples of fuels that can be liquefied at normal temperature in a pressurized state include propane, butane, and a mixture of these.

For example, a reaction formula (1) for the synthesis of methanol through catalytic hydrogenation of a source gas containing hydrogen and carbon dioxide in the presence of a catalyst is expressed as follows.

CO₂+3H₂ ⇔ CH₃OH+H₂O (1)

The above reaction is an equilibrium reaction, and the reactors 1 can shift the reaction equilibrium to the product side by separating water vapor, which is one of products of the conversion reaction. In order to increase the conversion efficiency and the reaction rate, the conversion reaction is preferably carried out at a high temperature and a high pressure (e.g., 180°C or higher and 2 MPa or higher). The liquid fuel is in a gaseous state when it is synthesized and remains in the gaseous state at least until the liquid fuel flows out from the reactors 1. It is preferable that the reactors 1 have heat resistance and pressure resistance suitable for synthesis conditions of the desired liquid fuel.

The arrangement and specific configuration of the reactors 1 will be described later.

### Housing 3

The housing 3 is mainly formed by a metallic material (e.g., stainless steel). The housing 3 houses the plurality of reactors 1. In the present embodiment, the external shape of the housing 3 is a circular columnar shape centered on a center axis AX. The center axis AX is an example of a "predetermined axis" according to the present invention.

The inside of the housing 3 is partitioned into first to fourth spaces P1 to P4 by the first sealing portion 4, the second sealing portion 5, and the flow stopper 6. The housing 3 includes a source gas supply port 3a, a liquid fuel discharge port 3b, a sweep gas supply port 3c, and a sweep gas discharge port 3d.

The source gas is supplied to the first space P1 via the source gas supply port 3a. The source gas flows into the reactors 1 from the first space P1. The liquid fuel flows out from the reactors 1 to the second space P2. The liquid fuel that has flown out to the second space P2 is discharged to the outside via the liquid fuel discharge port 3b.

The sweep gas is supplied to the third space P3 via the sweep gas supply port 3c. The sweep gas flows into the reactors 1 from the third space P3. The sweep gas that has taken in water vapor in the reactors 1 flows out to the fourth space P4 from the reactors 1. The sweep gas that has flown out to the fourth space P4 is discharged to the outside via the sweep gas discharge port 3d. The third space P3 is an example of a "non-outlet-side space" from which water vapor isolated in the reactors 1 does not flow out. The fourth space P4 is an example of an "outlet-side space" from which water vapor isolated in the reactors 1 flows out.

As shown in FIG. 1, the third space P3 is isolated from the fourth space P4 by the flow stopper 6. By providing the third space P3 and the fourth space P4 isolated from each other in the housing 3 in this manner, water vapor separated in the reactors 1 can be collected.

Note that, in the present embodiment, water vapor is collected using the sweep gas, but it is also possible to collect the water vapor without using the sweep gas by setting the pressure lower in the fourth space P4 than in the third space P3. In this case, a vacuum pump may be installed on the sweep gas discharge port 3d side.

### First Sealing Portion 4

The first sealing portion 4 seals a gap between the housing 3 and a first end portion 1a of each reactor 1. The first sealing portion 4 holds the first end portion 1a of each reactor 1. The first sealing portion 4 includes insertion holes into which the first end portions 1a of the reactors 1 are respectively inserted.

Examples of the constituent material for the first sealing portion 4 include glass, silver solder, solder, inorganic adhesive, rubber, and plastic.

### Second Sealing Portion 5

The second sealing portion 5 seals a gap between the housing 3 and a second end portion 1b of each of the reactors 1. The second sealing portion 5 holds the second end portion 1b of each reactor 1. The second sealing portion 5 includes insertion holes into which the second end portions 1b the reactors 1 are respectively inserted.

Since the second space P2 side of the second sealing portion 5 is exposed to a high temperature liquid fuel and water vapor, the constituent material for the second sealing portion 5 is required to have resistance to the chemical load of a high temperature liquid fuel and resistance to water vapor. Examples of the constituent material for the second sealing portion 5 include glass, silver solder, solder, and inorganic adhesive. Rubber and plastic are not suitable for the constituent material of the second sealing portion 5.

### Flow Stopper 6

The flow stopper 6 is disposed between the reactors 1 and the housing 3. The flow stopper 6 is disposed between the third space P3 and the fourth space P4. The flow stopper 6 isolates the third space P3 from the fourth space P4. The flow stopper 6 includes insertion holes into which the center portions of the reactors 1 are respectively inserted.

The flow stopper 6 suppresses the flow of the sweep gas from the third space P3 to the fourth space P4. Note that the flow stopper 6 only needs to suppress the flow of the sweep gas, and does not need to seal the space between the reactors 1 and the housing 3. The flow stopper 6 may be constituted of a material such as expanded graphite, rubber, or resin.

### Arrangement of Reactors 1

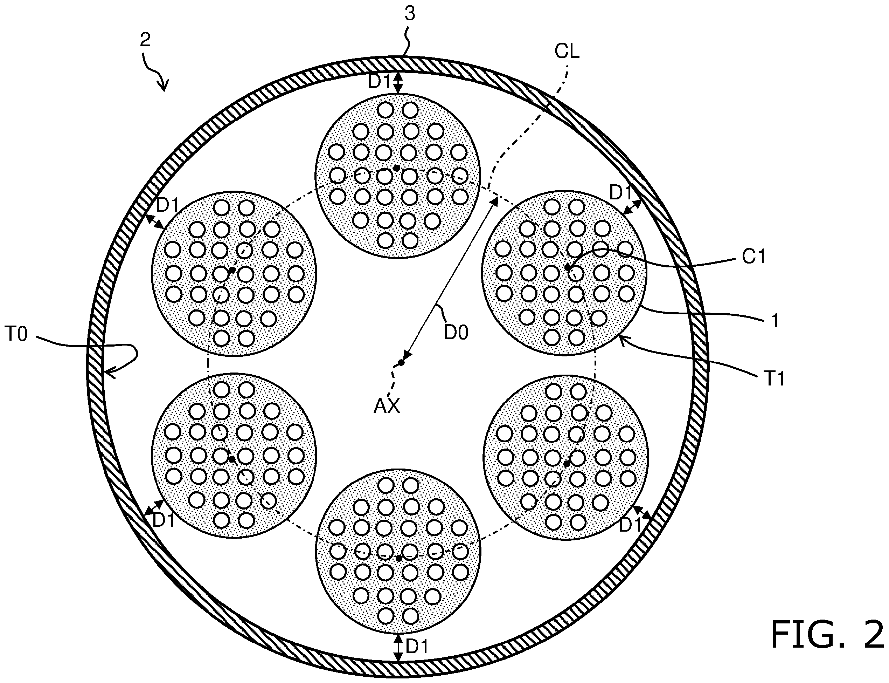

FIG. 2 is a cross-sectional view taken along line X-X in FIG. 1. FIG. 2 shows a cross section taken along a radial direction perpendicular to the center axis AX of the housing 3.

The plurality of reactors 1 are housed in the housing 3. Although the number of the reactors 1 needs to be at least two, in the present embodiment, as shown in FIG. 2, the case where six reactors 1 are provided will be described.

The six reactors 1 are arranged about the center axis AX in the housing 3. The reactors 1 are arranged on an analogous line CL that is analogous to the outline of an inner peripheral surface T0 of the housing 3. Specifically, a center C1 of a circular cross section of each reactor 1 is located on the analogous line CL. The analogous line CL is an example of a "one analogous line" according to the present invention. The reactors 1 are preferably arranged at equal intervals on the analogous line CL. Since six reactors 1 are provided in this embodiment, the interval between the centers C1 of the reactors 1 in the circumferential direction is 60 degrees.

Here, shortest distances D1 between the housing 3 and the reactors 1 are equivalent to each other. In other words, all the reactors 1 are spaced apart from the housing 3 by an equivalent distance. Accordingly, the reaction heat generated in the conversion reaction can be uniformly emitted from the reactors 1 to the outside via the housing 3, and thus the thermal environment of the reactors 1 can be made uniform. Accordingly, difference in the conversion efficiency and the lifetime of the reactors 1 can be suppressed.

The shortest distances D1 are distances between the inner peripheral surface T0 of the housing 3 and outer peripheral surfaces T1 of the reactors 1. In the present embodiment, the inner peripheral surface T0 of the housing 3 has a circular shape, and the outer peripheral surface T1 of each reactor 1 also has a circular shape.

"The shortest distances D1 are equivalent to each other" means not only a case where all the shortest distances D1 are the same value, but also a case where the difference between the maximum value and the minimum value included in all the shortest distances D1 is less than or equal to 30% of a distance D0. If the difference between the maximum and minimum shortest distances D1 is less than or equal to 30% of the distance D0, the thermal environment of the reactors 1 can be considered to be substantially uniform.

The distance D0 from the center axis AX to the analogous line CL is not particularly limited, but may be 81 mm or more and 874 mm or less, for example. The diameter of the inner peripheral surface T0 of the housing 3 is not particularly limited, but may be 351 mm or more and 2468 mm or less, for example. The diameter of the outer peripheral surface T1 of each reactor 1 is not particularly limited, but may be 90 mm or more and 360 mm or less, for example. The shortest distance D1 is not particularly limited, but may be 50 mm or more and 180 mm or less, for example.

### Detailed Configuration of Reactors 1

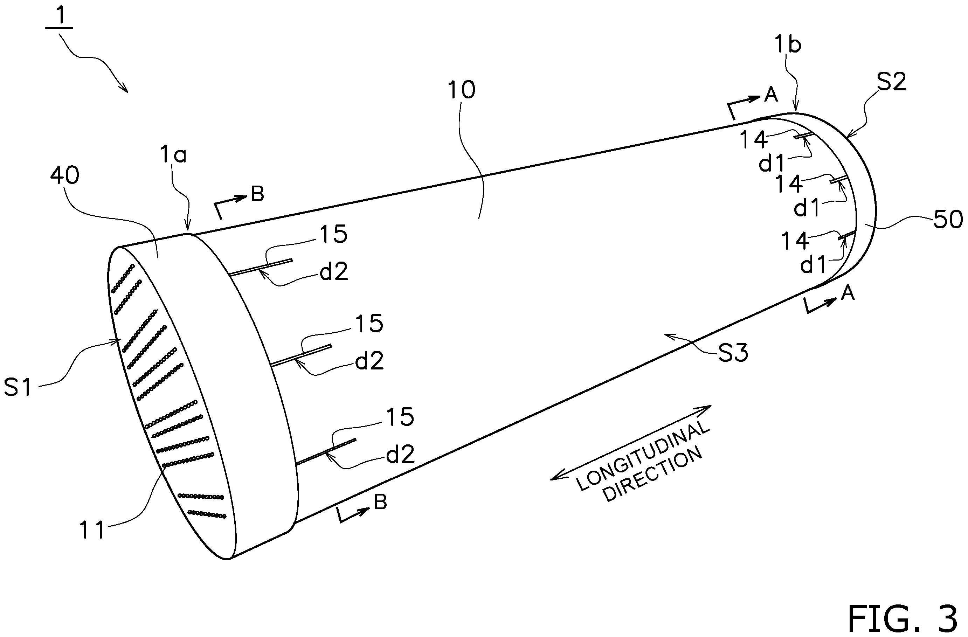

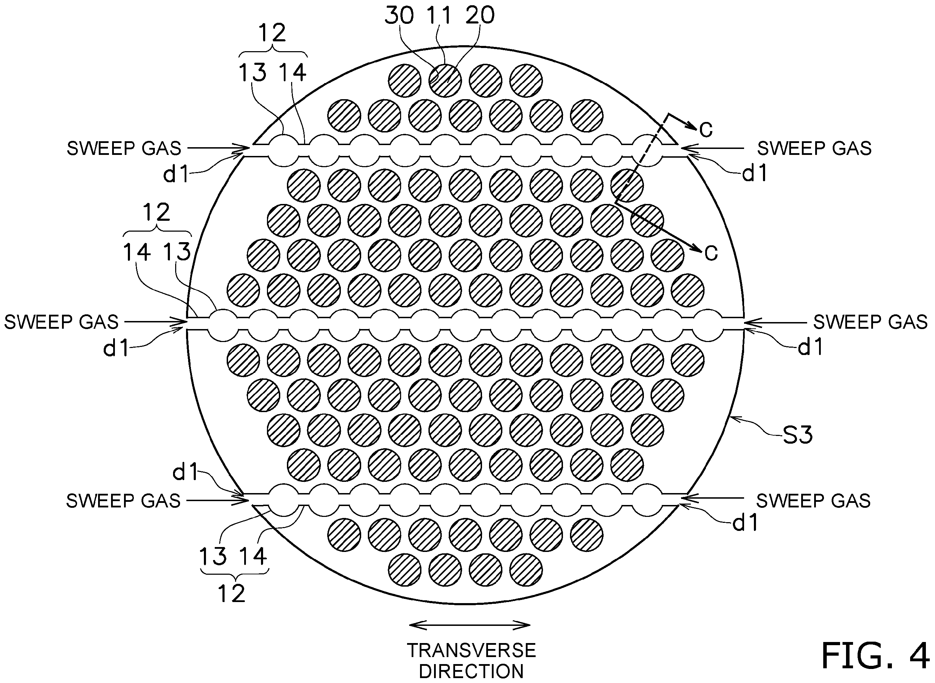

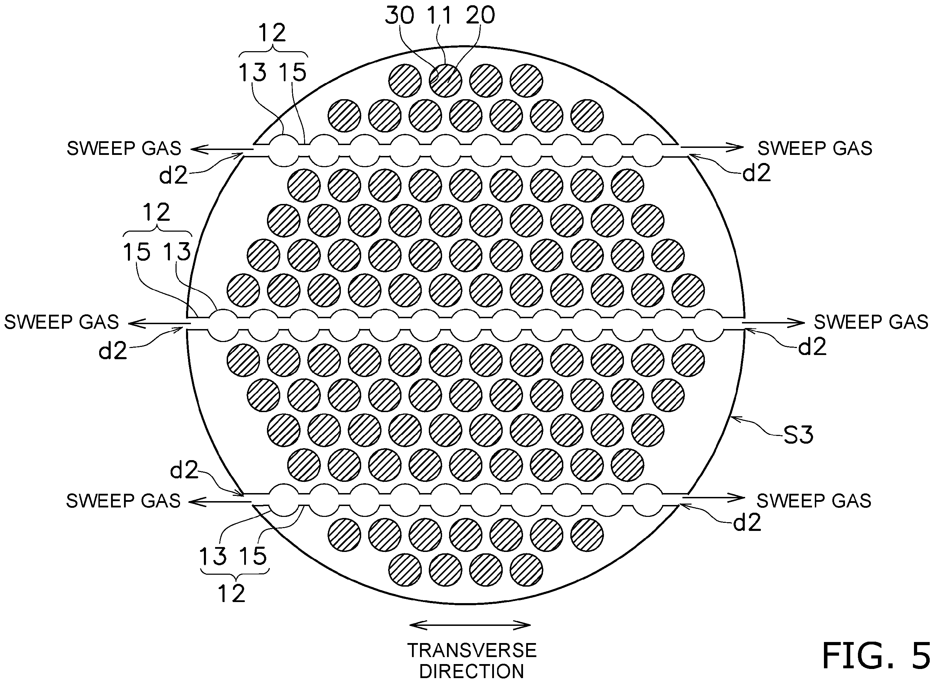

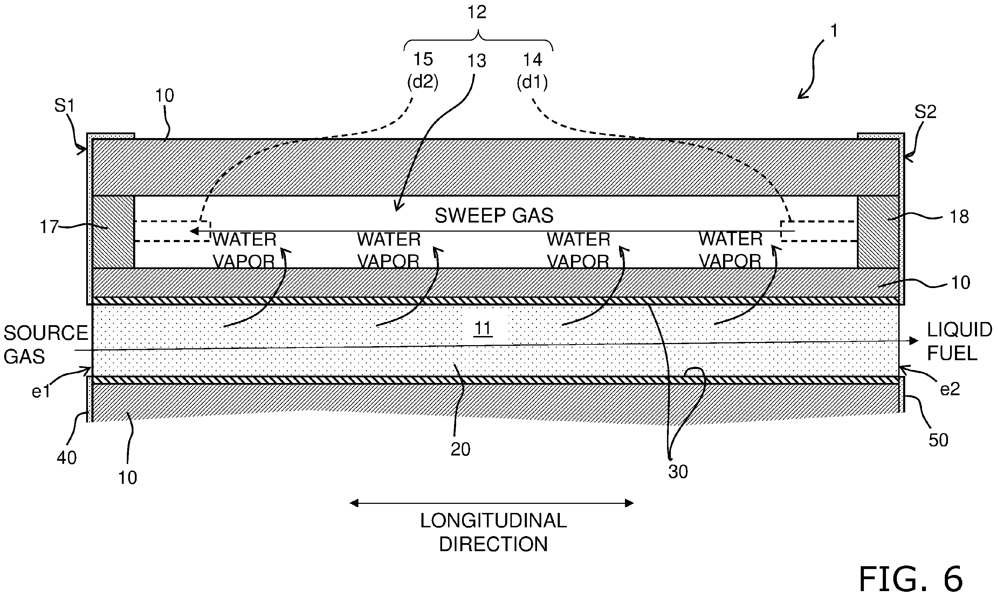

Next, the detailed configuration of the reactors 1 according to the present embodiment will be described. The above-described six reactors 1 have the same configuration. FIG. 3 is a perspective view of one of the reactors 1. FIG. 4 is a cross-sectional view taken along line A-A in FIG. 3. FIG. 5 is a cross-sectional view taken along line B-B in FIG. 3. FIG. 6 is a cross-sectional view taken along line C-C in FIG. 4.

As shown in FIG. 3, the reactor 1 has a monolith shape. The term "monolith" means a shape that includes a plurality of holes extending through the reactor 1 in the longitudinal direction, and encompasses a honeycomb shape. The longitudinal direction is a direction parallel with the center axis AX of the above-described housing 3.

The reactor 1 includes the first end portion 1a and the second end portion 1b. The first end portion 1a extends from an end of the reactor 1 in the longitudinal direction by a length of 2/5 of the entire length of the reactor 1 in the longitudinal direction. The second end portion 1b extends from the other end of the reactor 1 in the longitudinal direction by a length of 2/5 of the entire length of the reactor 1 in the longitudinal direction. In the present embodiment, the source gas flows into the reactor 1 from the first end portion 1a side, and the liquid fuel flows out of the reactor 1 from the second end portion 1b side.

The reactor 1 has a first end surface S1, a second end surface S2, and a side surface S3. The first end surface S1 is the end surface on the first end portion 1a side. The second end surface S2 is the end surface on the second end portion 1b side. The first end surface S1 is opposite to the second end surface S2. The side surface S3 is continuous to outer edges of the first end surface S1 and the second end surface S2.

As shown in FIGS. 3 to 5, the reactor 1 includes a porous support 10, a catalyst 20, a separation membrane 30, a first seal portion 40, and a second seal portion 50.

The porous support 10 is a circular columnar body extending in the longitudinal direction of the reactor 1. The porous support 10 is constituted by a porous material.

A ceramic material, a metallic material, a resin material, or the like can be used as the porous material, and a ceramic material is particularly favorable. At least one of alumina (Al₂O₃), titania (TiO₂), mullite (Al₂O₃·SiO₂), potsherd, and cordierite (Mg₂Al₄Si₅O₁₈) can be used as an aggregate for the ceramic material. At least one of titania, mullite, easily sinterable alumina, silica, glass frit, a clay mineral, and easily sinterable cordierite can be used as an inorganic binder for the ceramic material. However, the ceramic material need not necessarily contain an inorganic binder.

As shown in FIGS. 4 and 5, the porous support 10 includes a large number of first flow paths 11 and a plurality of second flow paths 12.

Each first flow path 11 is formed along the longitudinal direction of the reactor 1 as shown in FIG. 6. Each first flow path 11 is a space on a non-permeation side of the separation membrane 30. The source gas is caused to flow through the first flow paths 11. The first flow paths 11 are through holes. Each first flow path 11 is open in the first end surface S1 and the second end surface S2 of each reactor 1. Each first flow path 11 includes an inlet port e1 for the source gas formed in the first end surface S1 and an outlet port e2 for the liquid fuel formed in the second end surface S2. The catalyst 20 is disposed inside each first flow path 11. The number of first flow paths 11, their positions, shape, and the like can be changed as appropriate.

Each second flow path 12 is a space on a permeation side of the separation membrane 30. The sweep gas for sweeping water vapor that has passed through the separation membrane 30 is caused to flow through the second flow paths 12. Inert gas (e.g., nitrogen), air, or the like can be used as the sweep gas. The number of second flow paths 12, their positions, shape, and the like can be changed as appropriate.

As shown in FIGS. 4 and 5, each second flow path 12 is constituted by a plurality of cells 13, an inlet slit 14, and an outlet slit 15.

The plurality of cells 13 are lined up along a line extending in a transverse direction (a direction perpendicular to the longitudinal direction) of the reactor 1. Each cell 13 is formed along the longitudinal direction of the reactor 1 as shown in FIG. 6. Both ends of the cells 13 are sealed by first and second opening sealing portions 17 and 18. The first and second opening sealing portions 17 and 18 can be constituted by the porous material described above.

As shown in FIG. 3, the inlet slit 14 is formed in the second end portion 1b of the reactor 1. As shown in FIG. 4, the inlet slit 14 is formed along the transverse direction of the reactor 1. The inlet slit 14 extends through the plurality of cells 13. Both ends of the inlet slit 14 are open in the side surface S3. The inlet slit 14 includes a pair of inlet ports d1 that are open in the side surface S3. The pair of inlet ports d1 correspond to an end of the second flow path 12 in the longitudinal direction. The pair of inlet ports d1 are continuous with the third space P3. "The pair of inlet ports d1" is an example of a "first opening" according to the present invention.

As shown in FIG. 3, the outlet slit 15 is formed in the first end portion 1a of the reactor 1. As shown in FIG. 5, the outlet slit 15 is formed along the transverse direction of the reactor 1. The outlet slit 15 extends through the plurality of cells 13. Both ends of the outlet slit 15 are open in the side surface S3. The outlet slit 15 includes a pair of discharge ports d2 that are open in the side surface S3. The pair of discharge ports d2 correspond to the other end of the second flow path 12 in the longitudinal direction. The pair of discharge ports d2 are continuous with the fourth space P4. "The pair of discharge ports d2" is an example of a "second opening" according to the present invention.

The catalyst 20 is disposed inside each first flow path 11. The catalyst 20 preferably fills each first flow path 11, but may also be disposed so as to form a layer on the surface of the separation membrane 30. The catalyst 20 promotes the conversion reaction in which the source gas is converted into the liquid fuel, shown in the above formula (1).

A known catalyst suitable for the conversion reaction for obtaining the desired liquid fuel can be used as the catalyst 20. Examples of the catalyst 20 include metal catalysts (copper, palladium, etc.), oxide catalysts (zinc oxide, zirconia, gallium oxide, etc.), and composites of these (copper-zinc oxide, copper-zinc oxide-alumina, copper-zinc oxide-chromium oxide-alumina, copper-cobalt-titania, and these catalysts modified with palladium, etc.).

The separation membrane 30 is supported by the porous support 10. The separation membrane 30 surrounds the first flow paths 11. The separation membrane 30 is disposed between the first flow paths 11 and the second flow paths 12.

The separation membrane 30 is permeable to water vapor, which is one of the products generated as a result of the conversion reaction in which the source gas is converted into the liquid fuel. Thus, the reaction equilibrium of the above formula (1) can be shifted to the product side by utilizing an equilibrium shift effect.

It is preferable that the separation membrane 30 has a water vapor permeability coefficient of 100 nmol/(s·Pa·m²) or more. The water vapor permeability coefficient can be obtained using a known method (see Ind. Eng. Chem. Res., 40, 163-175(2001)).

It is preferable that the separation membrane 30 has a separation coefficient of 100 or more. The greater the separation coefficient, the easier it is for water vapor to pass through the separation membrane 30 and the more difficult it is for components (hydrogen, carbon dioxide, the liquid fuel, etc.) other than water vapor to pass through the separation membrane 30. The separation coefficient can be obtained using a known method (see FIG. 1 in "Separation and Purification Technology 239 (2020) 116533").

An inorganic membrane can be used as the separation membrane 30. Inorganic membranes have heat resistance, pressure resistance, and water vapor resistance and thus are preferable. Examples of inorganic membranes include zeolite membranes, silica membranes, alumina membranes, and composite membranes thereof. In particular, LTA-type zeolite membranes in which a molar ratio (Si/Al) between silicon element (Si) and aluminum element (Al) is 1.0 or more and 3.0 or less have excellent water vapor permeability and thus are favorable.

As shown in FIG. 3, the first seal portion 40 covers the first end surface S1 and a portion of the side surface S1 of the porous support 10. The first seal portion 40 suppresses intrusion of the source gas into the porous support 10. As shown in FIG. 6, the first seal portion 40 is formed so as not to close the inlet ports e1 of the first flow paths 11. The first seal portion 40 covers the first opening sealing portion 17. The first seal portion 40 can be constituted by glass, metal, rubber, resin, or the like.

As shown in FIG. 3, the second seal portion 50 covers the second end surface S2 and a portion of the side surface S1 of the porous support 10. The second seal portion 50 suppresses intrusion of the liquid fuel into the porous support 10. As shown in FIG. 6, the second seal portion 50 is formed so as not to close the outlet ports e2 of the first flow paths 11. The second seal portion 50 covers the second opening sealing portion 18. The second seal portion 50 can be constituted by glass, metal, rubber, resin, or the like.

### Method for Synthesizing Liquid Fuel Using Reactor 1

The following describes a method for synthesizing the liquid fuel using the reactor 1 with reference to FIG. 6.

The method for synthesizing the liquid fuel using the reactor 1 includes a step of causing the sweep gas to flow through the second flow paths 12 provided on the permeation side of the separation membrane 30 while causing the source gas to flow through the first flow paths 11 provided on the non-permeation side of the separation membrane 30.

The source gas flows into the first flow paths 11 from the inlet ports e1 of the first flow paths 11. In the first flow paths 11, water vapor is generated together with the liquid fuel as shown in the above formula (1). The synthesized liquid fuel flows out from the outlet ports e2 of the first flow paths 11. Water vapor, which is one of the products, passes through the separation membrane 30 and the porous support 10 in this order, and enters the second flow paths 12.

The sweep gas flows into the inlet ports d1 of the inlet slits 14 and then flows into the cells 13 from the inlet slits 14. Then, the sweep gas that has flowed into the cells 13 from the inlet slits 14 takes on water vapor that has passed through the separation membrane 30, and flows through the cells 13 toward the outlet slits 15 while absorbing reaction heat generated in the conversion reaction. The sweep gas that has reached the outlet slits 15 is discharged from the discharge ports d2 of the outlet slits 15.

As shown in FIG. 6, in the present embodiment, the direction of flows of the sweep gas in the second flow paths 12 is opposite to the direction of flows of the source gas in the first flow paths 11 in a side view of the separation membrane 30. That is, the sweep gas flows through the second flow paths 12 in a direction opposite to the direction in which the source gas flows through the first flow paths 11.

However, the direction of flows of the sweep gas in the second flow paths 12 may be the same as the direction of flows of the source gas in the first flow paths 11 in a side view of the separation membrane 30. That is, the sweep gas may flow through the second flow paths 12 in parallel to the source gas flowing through the first flow paths 11.

### 2. Second Embodiment

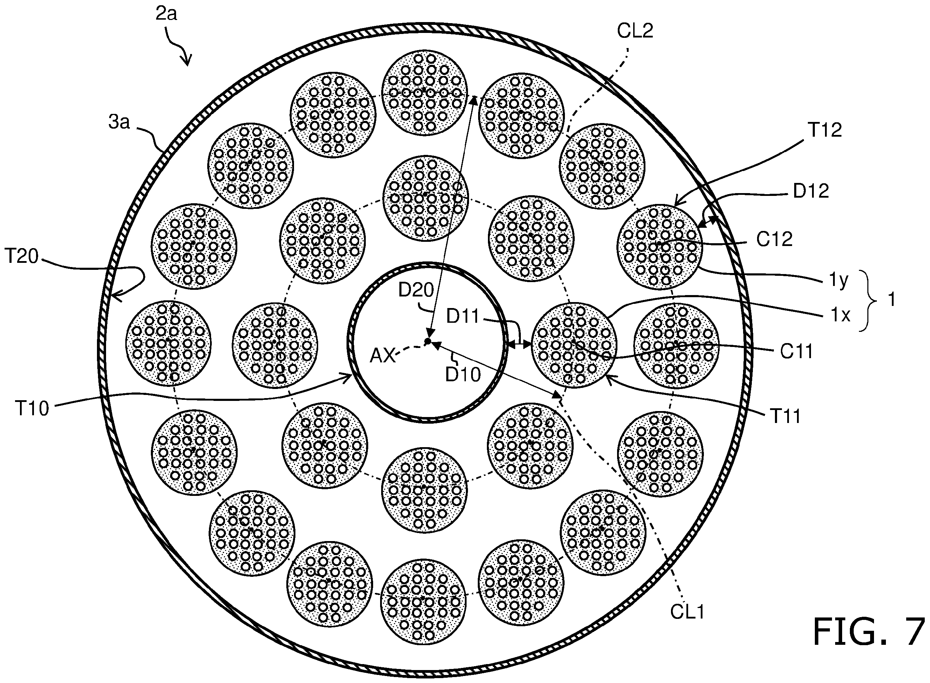

Next, a reactor module 2a according to a second embodiment will be described. FIG. 7 is a cross-sectional view of the reactor module 2a.

Since the reactor module 2a according to the second embodiment is different from the reactor module 2 according to the first embodiment in the configuration of the housing and the arrangement of the reactors, the following description will focus on the differences.

The reactor module 2a includes the plurality of reactors 1 and a housing 3a.

The plurality of reactors 1 are housed in the housing 3a. The configuration of the reactors 1 is as described in the first embodiment.

The plurality of reactors 1 include a plurality of first reactors 1x and a plurality of second reactors 1y. The plurality of first reactors 1x are arranged radially inside the plurality of second reactors 1y. The number of first reactors 1x only needs to be two or more, but in the present embodiment, as shown in FIG. 7, a case where eight first reactors 1x are provided will be described. Also, the number of second reactors 1y only needs to be two or more, but in the present embodiment, as shown in FIG. 7, a case where 16 second reactors 1y are provided will be described.

The eight first reactors 1x are arranged about the center axis AX in the housing 3a. The first reactors 1x are arranged on a first analogous line CL1 that is analogous to the outline of a first inner peripheral surface T10 or a second inner peripheral surface T20 of the housing 3. Specifically, a center C11 of a circular cross section of each first reactor 1x is located on the first analogous line CL1. The first reactors 1x are preferably disposed at equal intervals on the first analogous line CL1. Since the eight first reactors 1x are provided in the present embodiment, each interval in the circumferential direction between the centers C11 of the first reactors 1x is 45 degrees.

The 16 second reactors 1y are arranged about the center axis AX in the housing 3a. The second reactors 1y are arranged on a second analogous line CL2 that is analogous to the outline of the first inner peripheral surface T10 or the second inner peripheral surface T20 of the housing 3. Specifically, a center C12 of a circular cross section of each second reactor 1y is located on the second analogous line CL2. The second reactors 1y are preferably disposed at equal intervals on the second analogous line CL2. Since the 16 second reactors 1y are provided in the present embodiment, each interval in the circumferential direction between the centers C12 of the second reactors 1y is 22.5 degrees.

The external shape of the housing 3a is a circular columnar shape centered on the center axis AX. Note that the housing 3a has a hollow shape. Accordingly, the housing 3a is a hollow circular columnar body. The housing 3a includes the first inner peripheral surface T10 and the second inner peripheral surface T20. The first inner peripheral surface T10 is located radially inward of the second inner peripheral surface T20. The second inner peripheral surface T20 surrounds the first inner peripheral surface T10.

Here, the shortest distances between the housing 3a and the reactors 1 are equivalent to each other. Specifically, first shortest distances D11 between the housing 3a and the first reactors 1x are equivalent to each other, and second shortest distances D12 between the housing 3a and the second reactors 1y are equivalent to each other, and the first shortest distance D11 and the second shortest distance D12 are equivalent to each other. In other words, all the reactors 1 are spaced apart from the housing 3a by an equivalent distance.

For this reason, the reaction heat generated in the conversion reaction can be uniformly emitted from the reactors 1 to the outside via the housing 3a. Specifically, the reaction heat of the first reactors 1x is emitted to the hollow space of the housing 3a via the first inner peripheral surface T10, and the reaction heat of the second reactors 1y is emitted to the outer peripheral space of the housing 3a via the second inner peripheral surface T20. In this manner, the thermal environment of the reactors 1 can be made uniform, and thus generation of difference in the conversion efficiency and the lifespan of the reactors 1 can be suppressed.

The first shortest distance D11 is a distance between the first inner peripheral surface T10 of the housing 3a and an outer peripheral surface T11 of each first reactor 1x. In the present embodiment, the first inner peripheral surface T10 of the housing 3a has a circular shape, and the outer peripheral surface T11 of each first reactor 1x also has a circular shape.

The second shortest distance D12 is a distance between the second inner peripheral surface T20 of the housing 3a and an outer peripheral surface T12 of each second reactor 1y. In the present embodiment, the second inner peripheral surface T20 of the housing 3a has a circular shape, and the outer peripheral surface T12 of each second reactor 1y also has a circular shape.

In the present embodiment, the case in which the shortest distances are equivalent to each other encompasses not only the case where all the first and second shortest distances D11 and D12 are the same value, but also the case where the difference between the maximum and minimum first shortest distances D 11 and the difference between the maximum and minimum second shortest distances D12 are less than or equal to 15% of the distance D20. When the distance between the maximum and minimum first shortest distances D11 and the difference between the maximum and minimum second shortest distances D12 are less than or equal to 30% of the distance D20, the thermal environments of the reactors 1 can be considered to be substantially uniform.

The value of a distance D10 from the center axis AX to the first analogous line CL1 is not particularly limited, but may be 140 mm or more and 874 mm or less, for example. The value of the distance D20 from the center axis AX to the second analogous line CL2 is not particularly limited, but may be 280 mm or more and 1414 mm or less, for example. The value of the diameter of the first inner peripheral surface T10 of the housing 3a is not particularly limited, but may be 90 mm or more and 360 mm or less, for example. The value of the diameter of the second inner peripheral surface T20 of the housing 3a is not particularly limited, but may be 750 mm or more and 3548 mm or less, for example. The values of the first and second shortest distances D1 and D2 are not particularly limited, but may be 50 mm or more and 180 mm or less, for example.

### Modifications of Embodiments

Although embodiments of the present invention have been described, the present invention is not limited to the above embodiments, and various changes can be made within a scope not departing from the gist of the present invention.

### Modification 1

Although the first and second embodiments described that the external shape of the reactors1 and 1a is a circular columnar shape, the external shape may also be an elliptical columnar shape.

### Modification 2

Although the first and second embodiments described that the external shape of the housings 3 and 3a is a circular columnar shape, the external shape may also be an elliptical columnar shape.

### Modification 3

Although the first and second embodiments described that the inner peripheral surfaces of the housings 3 and 3a have a circular cross section, the housings 3 and 3a may also have an elliptical cross section. When the cross section of the inner peripheral surface of the housing has an elliptical shape, the analogous line also has an elliptical shape.

In the case where the plurality of reactors 1 are arranged on one analogous line as in the first embodiment and the analogous line has an elliptical shape, the case where the shortest distances are equivalent to each other encompasses not only a case where all the shortest distances are equivalent to each other, but also a case where the difference between the maximum and minimum shortest distances is less than or equal to 30% of the half length of the major axis of the analogous line.

In the case where the plurality of reactors 1 are arranged on a plurality of analogous lines as in the second embodiment and the analogous line has an elliptical shape, the case where the shortest distances are equivalent to each other encompasses not only a case where all the shortest distances are equivalent to each other, but also a case where the difference between the maximum and minimum shortest distances is less than or equal to 15% of the half length of the major axis of the longest analogous line.

### Modification 4

Although the first and second embodiments described that the outer peripheral surfaces of the reactors 1 have a circular cross section, the outer peripheral surfaces may also have an elliptical cross section.

### Modification 5

In the first and second embodiments, monolith type reactors 1 and 1a were described as an example of the reactors, but the configuration of the reactors is not limited to this. For example, the reactors may be reactors other than monolith type reactors. A representative example of reactors other than monolith type reactors is a tube type reactor (e.g., JP 2018-008940A), but there is no limitation to this. Note that, in tube-type reactors, the first flow paths 11 and the second flow paths 12 are open at the surfaces of the reactors (including both end surfaces and the side surface).

### Modification 6

Although the first and second embodiments described that the third space P3, which is an example of the "non-outlet-side space", and the fourth space P4, which is an example of the "outlet-side space" are isolated by the flow stopper 6, the separation structure of the "non-outlet-side space" and the "outlet-side space" is not particularly limited.

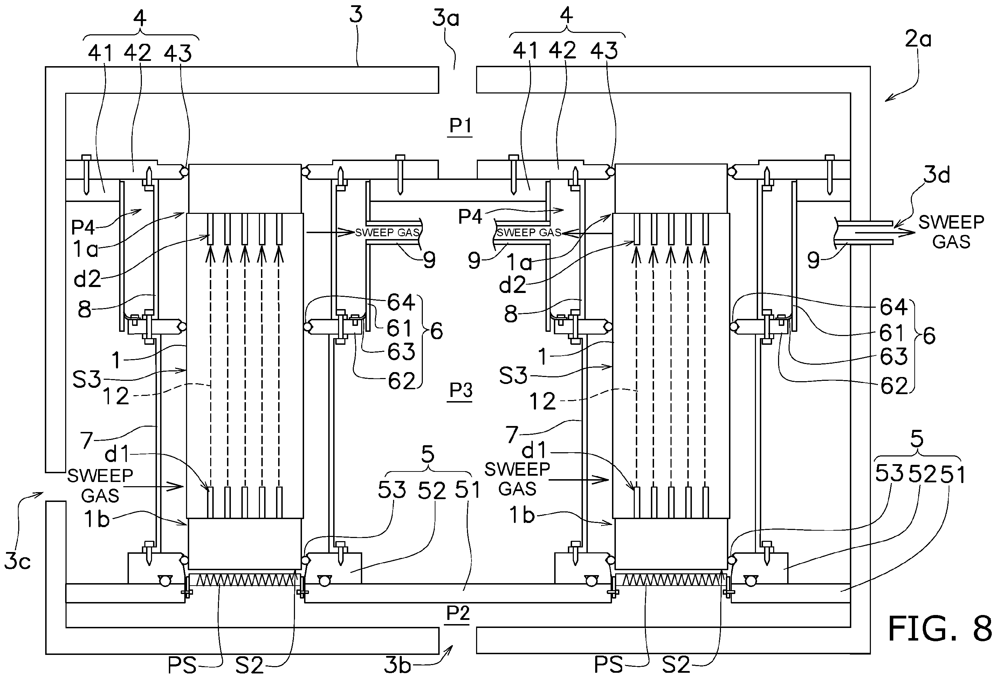

For example, as shown in FIG. 8, the "outlet-side space" isolated from the "non-outlet-side space" may be provided for each reactor 1. The reactor module 2a includes the plurality of reactors 1, the housing 3, first sealing portions 4, second sealing portions 5, flow stoppers 6, first flow resistance members 7, second flow resistance members 8, and a discharge pipe 9. Note that, in FIG. 8, members that are functionally similar to those in FIG. 1 are given the same reference signs.

The configuration of the reactors 1 is as described in the above embodiment.

The housing 3 is partitioned into the first to fourth spaces P1 to P4 by the first sealing portions 4, the second sealing portions 5, and the flow stoppers 6. The housing 3 includes the source gas supply port 3a, the liquid fuel discharge port 3b, the sweep gas supply port 3c, and the sweep gas discharge port 3d.

Each first sealing portion 4 seals a gap between the housing 3 and the first end portion 1a of the corresponding reactor 1. The first sealing portion 4 isolates the third space P3 and the fourth space P4 from the first space P1. The first sealing portions 4 are each constituted by a fixed plate 41, a flange 42 and a seal member 43. Note that, the flange 42 and the seal member 43 are provided for each reactor 1.

Each fixed plate 41 is connected to the inner peripheral surface of the housing 3. An opening for attaching the flow stopper 6 is provided in each fixed plate 41. The flanges 42 are annular members each fixed to an opening portion of the corresponding fixed plate 41. The seal members 43 have an annular shape and surround the first end portion 1a of the corresponding reactor 1. The seal members 43 each seal a gap between the flange 42 and the first end portion 1a of the corresponding reactor 1. An O-ring or the like may be used as the seal members 43, for example.

Each second sealing portion 5 seals a gap between the housing 3 and the second end portion 1b of the corresponding reactor 1. Each second sealing portion 5 isolates the third space P3 and the fourth space P4 from the second space P2. The second sealing portions 5 are each constituted by a fixed plate 51, and a flange 52, and a seal member 53. Note that, the flange 52 and the seal member 53 are provided for each reactor 1.

The fixed plates 51 are connected to the inner peripheral surface of the housing 3. An opening for attaching the flange 52 is formed in each fixed plate 51. The flanges 52 are annular members respectively fixed to the openings of the fixed plates 51. The seal members 53 each have an annular shape and surround the second end portion 1b of the corresponding reactor 1. The seal members 53 each seal a gap between the flange 52 and the second end portion 1b of the corresponding reactor 1. An O ring or the like may be used as the seal members 53, for example.

The flow stoppers 6 are disposed between the third space P3 and the fourth spaces P4. The flow stoppers 6 each partition the third space P3 and the fourth space P4. The flow stoppers 6 are each formed by a partition pipe 61, a partition plate 62, a packing 63, and a seal member 64. Note that a flow stopper 6 is provided for each reactor 1.

The partition pipes 61 are tubular members each attached to the opening in the corresponding fixed plate 41. The partition pipes 61 are each disposed so as to surround the discharge port d2 of the corresponding reactor 1. The partition plates 62 are annular members each disposed between the reactor 1 and the corresponding partition pipe 61. The packings 63 are annular elastic members each fixed to an outer periphery of the partition plate 62. The packing 63 seals a gap between the partition pipe 61 and the partition plate 62. The seal members 64 each seal a gap between the reactor 1 and the corresponding partition plate 62. An O ring or the like may be used as the seal members 64, for example.

The first flow resistance members 7 are tubular members each surrounding the inlet port d1 of the corresponding reactor 1. The two end portions of each first flow resistance member 7 are respectively fixed to the partition plate 62 and the flange 52. The first flow resistance member 7 imparts flow resistance to the sweep gas flowing from the third space P3 into the inlet port d1 of the reactor 1. In this manner, since pressure loss is generated in the sweep gas supplied from the third space P3, regardless of the distance from the sweep gas supply port 3c, the amount of the sweep gas flowing into the inlet port d1 of each reactor 1 can be made uniform. As a result, since the reactors 1 can be uniformly cooled, the thermal environment of the reactors 1 can be made even more uniform.

The second flow resistance members 8 are tubular members that each surround the outlet port d2 of the corresponding reactor 1. The two end portions of each second flow resistance member 8 are respectively fixed to the partition plate 62 and the flange 42. The second flow resistance members 8 each impart flow resistance to the sweep gas (including water vapor) flowing out from the outlet port d2 of the reactor 1 to the fourth space P4. In this manner, since pressure loss is generated in the sweep gas flowing out from the outlet ports d2, regardless of the distance from the collection pipe 93 (described later), the flow amount of the sweep gas flowing through the second flow path 12 of each reactor 1 can be made uniform. As a result, since the reactors 1 can be uniformly cooled, the thermal environment of the reactors 1 can be made even more uniform.

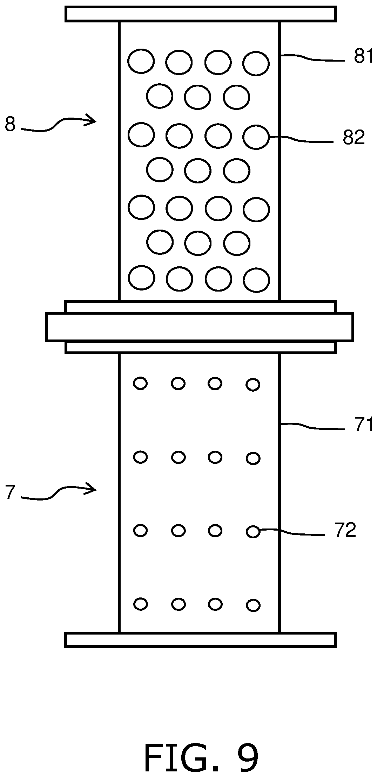

Here, FIG. 9 is a side view of the first flow resistance member 7 and the second flow resistance member 8. The first flow resistance member 7 includes a tube body 71 and a plurality of ventilation holes 72. The ventilation holes 72 function as orifices. The flow resistance of the first flow resistance member 7 can be adjusted by the total opening area that is determined in accordance with the size and number of ventilation holes 72. The second flow resistance member 8 includes a tube body 81 and a plurality of ventilation holes 82. The ventilation holes 82 function as orifices. The flow resistance of the second flow resistance member 8 can be adjusted by the total opening area that is determined in accordance with the size and number of ventilation holes 82.

As shown in FIG. 9, the total opening area of the first flow resistance member 7 is smaller than the total opening area of the second flow resistance member 8. Accordingly, the flow resistance of the first flow resistance member 7 is larger than the flow resistance of the second flow resistance member 8. Accordingly, since a large flow resistance can be provided to the sweep gas that flows from the third space P3 into the inlet port d1, regardless of the distance from the sweep gas supply port 3c, the flow amount of the sweep gas flowing to the inlet port d1 of each reactor 1 can be made uniform. As a result, the reactors 1 can be uniformly cooled, and thus the thermal environment of the reactors 1 can be made even more uniform.

Note that the reactor module 2a does not need to include one of or both the first flow resistance member 7 and the second flow resistance member 8. Also, the flow resistance of the second flow resistance member 8 may also be larger than the flow resistance of the first flow resistance member 7.

The discharge pipe 9 is connected to each partition pipe 61. The discharge pipe 9 discharges the sweep gas (including water vapor) that has flowed out from the outlet port d2 of the reactor 1 to the fourth space P4 to the outside.

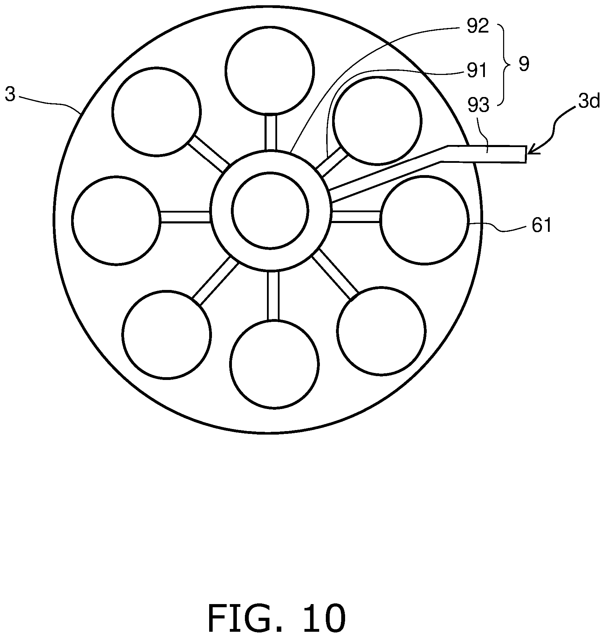

Here, FIG. 10 is a top view schematically showing the configuration of the discharge pipe 9. As shown in FIG. 10, the discharge pipe 9 includes a plurality of small pipes 91, an annular pipe 92, and a collection pipe 93. The small pipes 91 are respectively in communication with the partition pipes 61 and the annular pipe 92. The annular pipe 92 is in communication with the small pipes 91 and the collection pipe 93. The collection pipe 93 passes through the housing 3. The sweep gas discharge port 3d is formed at a distal end of the collection pipe 93. By the sweep gas that has flown out from the reactors 1 being collected to the one collection pipe 93 to be discharged to the outside in this manner, the seal of the housing 3 can be easily maintained. Note that the configuration of the discharge pipe 9 is not limited to that shown in FIG. 10.

Note that, as shown in FIG. 8, the reactor module 2a may also include a packing support PS. The packing support PS suppresses flowing-out of the catalyst 20 from the first flow paths 11 (see FIG. 6) of the reactor 1. The packing support PS covers the second end surface S2 on the second end portion 1b side of the reactor 1.

Although specific examples of the partition structure between the third space P3, which is the example of the "non-outlet-side space", and the fourth space P4, which is an example of the "outlet-side space", have been described above, the shape and function of the members may be changed as appropriate. In addition, although the collection of water vapor is performed using the sweep gas in the present modification as well, the water vapor may be collected by setting the pressure lower in the fourth space P4 than in the third space P3, without using the sweep gas. Also, the sweep gas may flow in the order of the fourth space P4, the second flow path 12, and the third space P3 (i.e., the order opposite to the flow of the sweep gas shown in FIG. 8).

### Modification 7

In the first and second embodiments, and Modifications 1 to 6, the separation membrane 30 is permeable to water vapor, which is one of products of the conversion reaction in which the source gas is converted into the liquid fuel, but the present invention is not limited to this. The separation membrane 30 may also be permeable to the liquid fuel generated as a result of the conversion reaction in which the source gas is converted into the liquid fuel. In this case as well, it is possible to shift the reaction equilibrium of the above formula (1) to the product side.

Also, in the case where the separation membrane 30 is permeable to the liquid fuel, the reaction equilibrium can be shifted to the product side even when the liquid fuel is generated as a result of a reaction in which no water vapor is generated (e.g., 2H₂+CO ⇄ CH₃OH).

### Modification 8

In the present embodiment, a reactor module having a reactor has been described. However, the present invention can be applied to a separation membrane module including a plurality of separation filters that require heating or cooling. The separation filters each include a separation membrane for separating a predetermined component from a mixed fluid.

In this separation membrane module, in order to develop or maintain separation membrane performance, the separation membrane may be heated or cooled to be used at a temperature different from normal temperature. In such a case, similarly to the above embodiment, by making the shortest distances between the housing and the separation filters equivalent to each other, the separation filters can be uniformly heated or cooled from the outside, and thus the thermal environment of the separation filters can be made uniform. Accordingly, generation of difference in the separation performance and lifespan of the separation filters can be suppressed.

For example, Non-Patent Literature (Microporous and Mesoporous Materials 132 (2010) 137-147) discloses that the gas permeation performance of the DDR-type zeolite membrane depends on the temperature. Also, when a ceramic separation filter is heated or cooled more than expected, thermal stress may be generated at a separation layer interface, which may lead to material failure. Accordingly, if there is a separation filter that is not sufficiently heated or cooled, or a separation filter that is excessively heated or cooled, the separation performance may relatively deteriorate, or the life span may be shorter. For this reason, it is important to uniformly set the thermal environments of the separation filters to a desired temperature.

Note that methods for heating or cooling the separation filter include a method in which the housing is heated or cooled from the outside, and a method in which fluid that has been heated or cooled is caused to flow into the separation filter, for example, but there is no limitation to this.

### REFERENCE SIGNS LIST

- 1: Reactor
- 1x: First reactor
- 1y: Second reactor
- 2, 2a: Reactor module
- 3, 3a: Housing
- AX: Center axis
- CL: Analogous line
- CL1: First analogous line
- CL2: Second analogous line

## Claims

1. A reactor module comprising:
a housing; and
a plurality of reactors arranged about a predetermined axis in the housing, wherein
in a cross section along a radial direction perpendicular to the predetermined axis, shortest distances between the housing and the plurality of reactors are equivalent to each other.

2. The reactor module according to claim 1, wherein
the plurality of reactors are arranged on one analogous line analogous to an outline of an inner peripheral surface of the housing.

3. The reactor module according to claim 1, wherein
the housing is hollow, and
the plurality of reactors include a plurality of first reactors arranged on a first analogous line analogous to an inner peripheral surface of the housing, and a plurality of second reactors arranged on a second analogous line analogous to the inner peripheral surface of the housing and surrounding the first analogous line.

4. The reactor module according to any one of claims 1 to 3, wherein
an external shape of the housing is a circular columnar shape or an elliptical columnar shape centered on the predetermined axis, and
an external shape of each of the plurality of reactors is a circular columnar shape or an elliptical columnar shape.

5. The reactor module according to claim 1, wherein
the reactors each include a separation membrane permeable to a product of conversion reaction for converting a source gas containing hydrogen and carbon oxide into a liquid fuel, a first flow path located on non-permeation side of the separation membrane, and a second flow path located on permeation side of the separation membrane,
the second flow path includes a first opening and a second opening in a surface of the reactor,
the first opening is in communication with a non-outlet-side space in the housing,
the second opening is in communication with an outlet-side space in the housing, and
the non-outlet-side space is isolated from the outlet-side space.

6. The reactor module according to claim 5, further comprising:
first ventilation resistance member disposed in the non-outlet-side space and surrounding the first opening of the reactor, wherein
the first flow resistance member provides flow resistance to a sweep gas flowing into the first opening from the non-outlet-side space.

7. The reactor module according to claim 6, further comprising:
second flow resistance member disposed in the outlet-side space and surrounding the second opening of the reactor, wherein
the second flow resistance member provides flow resistance to a sweep gas flowing out from the second opening to the outlet-side space, and
a flow resistance of either one of the first flow resistance member and the second flow resistance member is larger than a flow resistance of another one of the first flow resistance member and the second flow resistance member.

8. A separation membrane module comprising:
a housing; and
a plurality of separation filters arranged about a predetermined axis in the housing and requiring heating or cooling, wherein
in a cross section taken along a radial direction perpendicular to the predetermined axis, shortest distances between the housing and the plurality of separation filters are equivalent to each other.
